# EUROPEAN PATENT SPECIFICATION

(11) **EP 2 500 003 B1**
(45) Date of publication and mention of the grant of the patent: **02.09.2020**
(21) Application number: 10829943.9
(22) Date of filing: 10.11.2010
(51) Int. Cl.: A61F 13/49, A61F 13/15, A61F 13/472, A61F 13/494, A61F 13/53

(54) **ABSORPTIVE ARTICLE**
SAUGFÄHIGER ARTIKEL
ARTICLE ABSORBANT

(30) Priority: 13.11.2009 JP 2009260483
(43) Date of publication of application: 19.09.2012
(73) Proprietor: Unicharm Corporation, Ehime 799-0111 (JP)
(72) Inventor: KAWAMURA, Koji, Kanonji-shi Kagawa 769-1602 (JP); KURITA, Noriyuki, Kanonji-shi Kagawa 769-1602 (JP)
(74) Representative: Dolleymores
(86) International application number: PCT/JP2010/069973
(87) International publication number: WO 2011/058984

(56) References cited:
- EP-A1- 1 776 940
- EP-A1- 2 554 148
- WO-A1-92/10984
- WO-A1-96/12457
- JP-A- 1 162 802
- JP-A- 2001 340 369
- JP-A- 2005 335 393
- JP-A- 2006 095 156
- JP-A- 2009 028 186
- US-A- 4 846 815
- US-A1- 2006 069 371

## Description

### {Technical Field}

The present disclosure relates to absorbent articles and, more particularly, to absorbent articles such as urine absorption pads, disposable diapers, toilet-training pants, incontinence briefs and the like.

### {Background}

Absorbent articles including a topsheet, a backsheet and an absorbent member interposed between these sheets are known. For example, PTL 1 discloses an absorbent article including an absorbent member formed with through holes so that bodily fluids such as urine may pass downward through the through holes. In such an absorbent article, urine discharged on the topsheet may be absorbed on the topsheet side of the absorbent member and urine having passed downward through the through holes to the backsheet side may be absorbed also on the backsheet side of the absorbent member. In this way, it is possible for such an absorbent article to absorb urine over a wider area than the case in which urine discharged on the topsheet is absorbed by the absorbent member only on one side of the absorbent member.

### {Citation List}

### {Patent Literature}

PTL 1: JP 2008-284190 A
US 2006/0069371 A1 discloses a disposable absorbent pad comprising an absorbent fibrous structure having a first surface and a second surface: a linear through-hole extending in the first surface has length and width dimensions different from the length and width dimensions of the linear through-hole extending in the second surface.
{Summary of Invention}

### {Technical Problem}

In the above-mentioned through holes, an opening area on the side of the topsheet is substantially the same as an opening area on the side of the backsheet and a cross-sectional surface of the through holes is substantially perpendicular to the absorbent member. Bodily fluids may be absorbed not only on the topsheet side but also on the backsheet side of the absorbent member. However, it cannot be found from the disclosure of PTL 1 that the cross-sectional surface also may be used to absorb bodily fluids.

The present invention provides an absorbent article adapted to accelerate the absorption rate of the absorbent member, to enlarge the absorption area in the absorbent member and thereby to improve the absorption capacity of the absorbent member.

### {Solution to Problem}

According to this invention, there is provided the absorbent article of independent claim 1. The dependent claims specify preferred but optional features.

An absorbent article has a longitudinal direction and a transverse direction and including a topsheet lying on a side facing the wearer's body, a backsheet lying on a side opposite to the side facing the wearer's body and an absorbent member interposed between the topsheet and the backsheet, wherein the absorbent member is formed with a through hole extending through the absorbent member from an upper surface thereof lying on the side of the topsheet to a lower surface thereof lying on the side of the backsheet.

In the above-mentioned absorbent article, the through hole has an upper opening on the upper surface, a lower opening on the lower surface and a peripheral wall connecting the upper opening to the lower opening, and at least one of the front and rear ends in the longitudinal direction of the upper opening is not aligned in the thickness direction with front or rear end in the longitudinal direction of the lower opening and the peripheral wall extends down- and inward from the front and/or rear end in the longitudinal direction of the upper opening to the front and/or rear end of the lower opening.

According to one embodiment of this invention, an opening area of the upper opening is larger than that of the lower opening and a dimension in the longitudinal direction of the upper opening is larger than that of the lower opening.

According to another embodiment of this invention, one of the front and rear ends of the lower opening extends outward in the longitudinal direction beyond the corresponding end of the upper opening.

According to yet another embodiment of this invention, the through hole has a dimension in the longitudinal direction larger than the dimension thereof in the transverse direction.

According to still another embodiment of this invention, the peripheral wall is formed with a stepped region created by a thickness dimension difference of the peripheral wall and extending in a circumferential direction.

According to the present invention, upper and lower surfaces of the absorbent member are covered with liquid diffusion sheets, respectively.

According to the present invention, the absorbent member includes a plurality of layer elements stacked in the thickness direction and a liquid diffusion sheet is interposed between each pair of the adjacent layer elements.

According to the present invention, the absorbent member is formed with compressed grooves extending in the longitudinal direction outboard of the through hole as viewed in the transverse direction.

According to the present invention, between the lower surface of the absorbent member and the backsheet, there are provided two or more bonding means spaced apart from each other in the transverse direction and extending in the longitudinal direction.

### {Advantageous Effects of Invention}

According to this invention, the through hole formed in the absorbent member has a peripheral wall extending down- and inward from the front and/or rear end of the upper opening to the front and/or rear end of the lower opening so that the area of the peripheral wall exposed to the upper opening may be enlarged in comparison with a case in which the peripheral wall vertically extends between the upper and lower openings. Correspondingly, it is possible to enlarge the absorption area in the absorbent member, to accelerate the absorption rate of the absorbent member, and to improve the absorption capacity of the absorbent member.

In addition, the peripheral wall extending down- and inward from the front and/or rear end of the upper opening to the front and/or rear end of the lower opening has a thickness smaller than the remaining region and advantageously provides a relatively high flexibility along fold lines extending in the longitudinal direction so that, even when the absorbent article is folded along these fold lines, such a high flexibility functions to prevent the opposite side edges of the lower opening from coming in contact with each other and closing the opening.

### {Brief Description of Drawings}

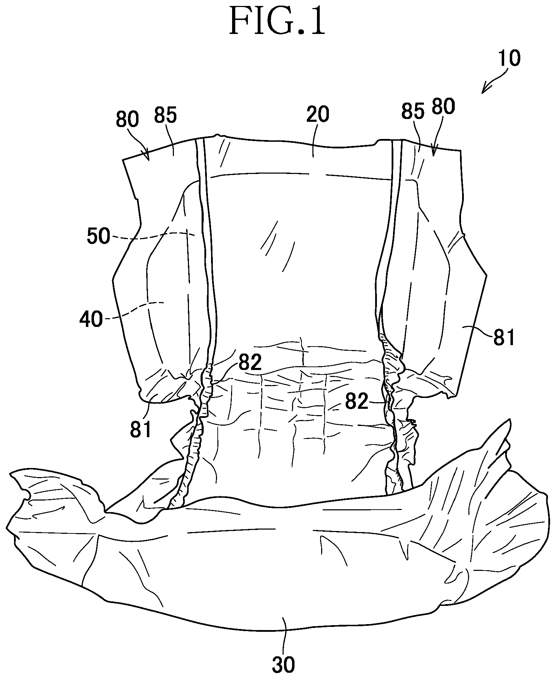

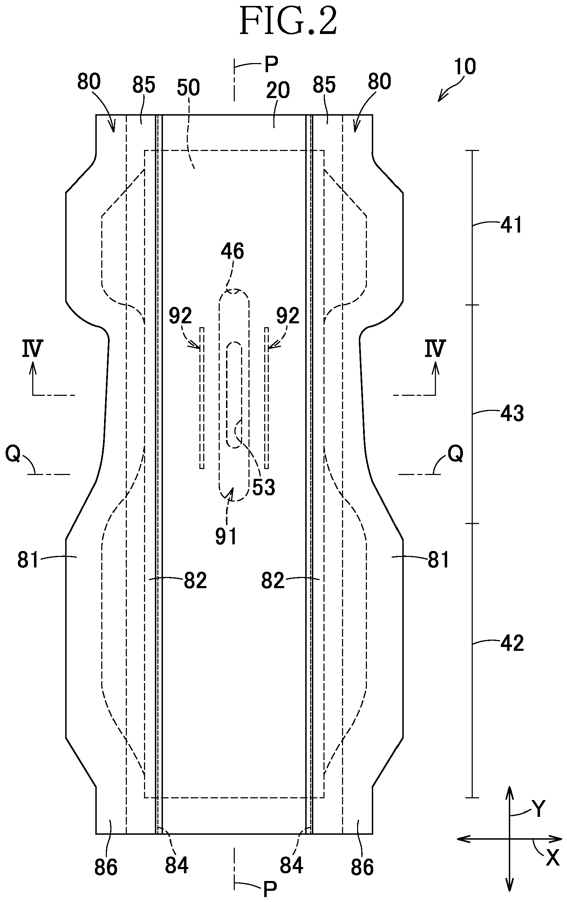

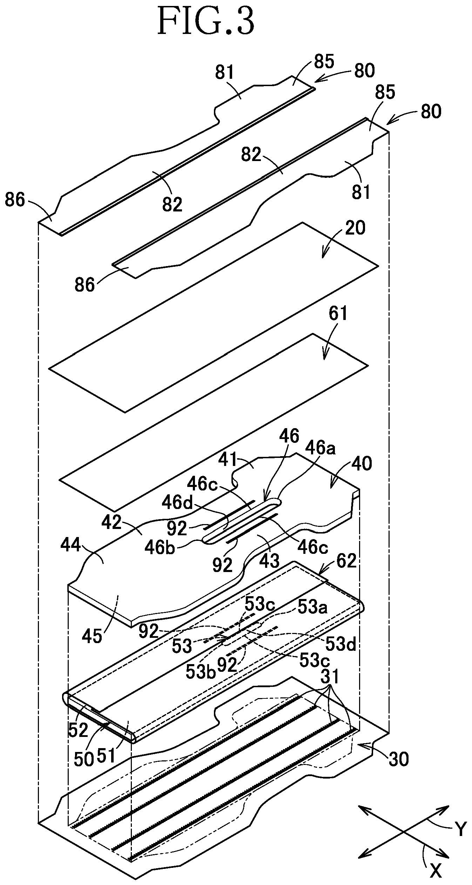

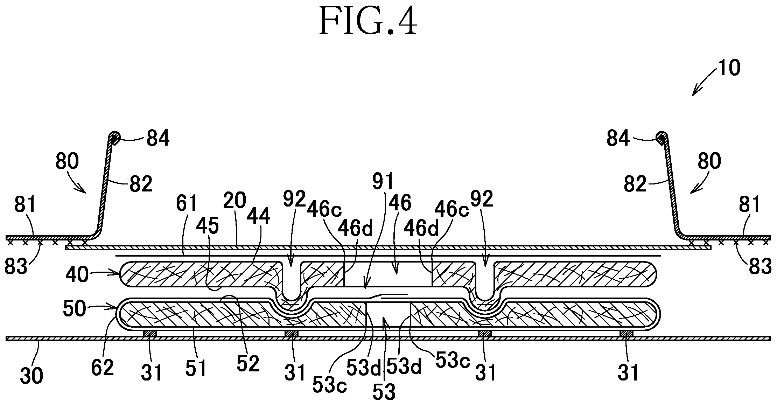

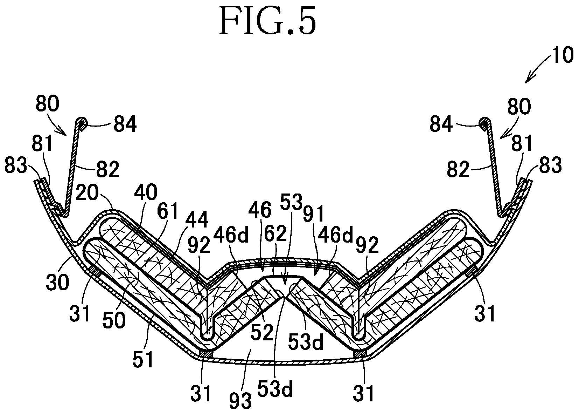

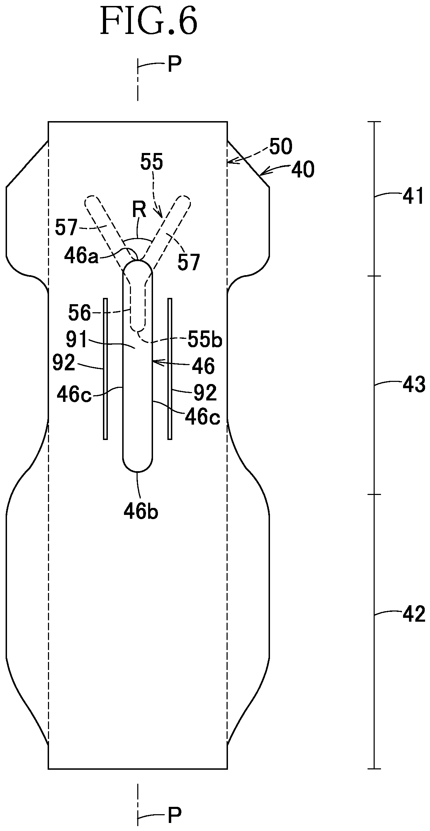

{Fig. 1} Fig. 1 is a perspective view of a urine absorption pad as one example of the absorbent article.
{Fig. 2} Fig. 2 is a flat plan view of a first embodiment.
{Fig. 3} Fig. 3 is an exploded view of Fig. 2.
{Fig. 4} Fig. 4 is a sectional view taken along line IV-IV in Fig. 2.
{Fig. 5} Fig. 5 is a diagram illustrating Fig. 4 in a state put on the wearer's body.
{Fig. 6} Fig. 6 is a plan view of the absorbent article according to a second embodiment.

### {Description of Embodiments}

### <First Embodiment>

Figs. 1 through 5 illustrate a first embodiment of this invention and, referring to Figs. 1 through 5, the absorbent article according to this invention will be described hereunder on the basis of a urine absorption pad as one example thereof.

Fig. 1 is a perspective view of a urine absorption pad 10, Fig. 2 is a flat plan view of the urine absorption pad 10 as viewed from the side of the wearer's body wherein elastic members are stretched against contractile force thereof to keep the urine absorption pad 10 in a flat state, Fig. 3 is an exploded view of Fig. 2, Fig. 4 is a sectional view taken along line IV-IV in Fig. 2 and Fig. 5 is a sectional view similar to Fig. 4, illustrating the sectional view when the urine absorption pad 10 is put on the wearer's body. In this regard, the respective sectional views are schematic diagrams in which respective sheets are illustrated so as to be spaced apart from each other in order to clarify the manner in which respective sheets are stacked. In Fig. 4, elastic members 84 for leakage-barrier cuffs 80 are illustrated in a contraction state thereof. The urine absorption pad 10 includes an imaginary longitudinal center line P-P bisecting a dimension in a transverse direction X and an imaginary transverse center line Q-Q bisecting a dimension in a longitudinal direction Y and an outer shape of the urine absorption pad 10 is substantially symmetric about the imaginary longitudinal center line P-P.

The urine absorption pad 10 has a longitudinal direction Y and a transverse direction X and includes a topsheet 20 lying on the side of the wearer's body, a backsheet 30 lying on the side opposite to the topsheet 20, i.e., on the side of the wearer's garment and an absorbent member interposed between the topsheet 20 and the backsheet 30. The topsheet 20 is liquid-permeable and may be formed, for example, of a polypropylene spun bonded fiber nonwoven fabric having a mass per unit area of about 20g/m² and hydrophilized. The backsheet 30 is liquid-impermeable and may be formed, for example, of a polyethylene film having a mass per unit area of about 23.5g/m² and water repellent finished.

The absorbent member includes a first layer element 40 stacked in the thickness direction and lying on the side of the topsheet 20 and a second layer element 50 stacked in the thickness direction and lying on the side of the backsheet 30. The first and second layer elements 40, 50 respectively include a liquid-absorbent core material formed of for example absorbent pulp and/or superabsorbent polymer particles.

A first liquid diffusion sheet 61 is interposed between the first layer element 40 and the topsheet 20 and upper and lower surfaces of the second layer element 50 are wrapped with a second liquid diffusion sheet 62. As the first and second liquid diffusion sheets 61, 62, for example, tissue paper or a fibrous nonwoven fabric having a mass per unit area of about 15g/m² may be used.

The topsheet 20 is provided on the side of the wearer's body with a pair of leakage-barrier cuffs 80 symmetrically about the imaginary longitudinal center line P-P so as to be spaced apart from each other in the transverse direction X. Respective outer edges 81 in the transverse direction X of the leakage-barrier cuffs 80 are bonded to the topsheet 20 and the backsheet 30 by bonding means 83 such as hot melt adhesives. Respective inner side edges 82 are not bonded to the topsheet 20 so that the inner side edges 82 may be spaced apart from the topsheet 20 (See Fig. 4) . The respective inner side edges 82 form sleeves and elastic members 84 are attached within these sleeves.

The elastic members 84 extend in the longitudinal direction Y and are attached under tension and in a contractible manner. Front and rear ends 85, 86 of the respective leakage-barrier cuffs 80 are bonded to the topsheet 20 and the backsheet 30 by bonding means such as hot melt adhesives (not shown) . With the urine absorption pad 10 put on the wearer's body, the inner side edges 82 are spaced upward from the topsheet 20 under contraction of the elastic members 84 and thereby may prevent bodily waste such as urine from leaking out beyond the opposite side edges of the urine absorption pad 10. As the leakage-barrier cuffs 80, for example, a spun bonded/melt blown/spun bonded (SMS) nonwoven fabric made of polypropylene fibers having a mass per unit area of about 15g/m² may be used.

The first layer element 40 has front and rear sections 41, 42 spaced apart from each other in the longitudinal direction Y and a constricted section 43 extending between these front and rear sections 41, 42 and bent or curved in the vicinity of the imaginary transverse center line Q-Q so that a dimension thereof in the transverse direction X is reduced. The first layer element 40 and the backsheet 30 are substantially similar in outer shapes thereof but the backsheet 30 is sized to be larger than the first layer element 40. The first layer element 40 has an upper surface 44 facing the topsheet 20 and a lower surface 45 facing the second layer element 50 and the first layer element 40 is formed with a slot-like first through hole 46 extending nearly vertically in the thickness direction from the upper surface 44 to the lower surface 45.

The first through hole 46 lies at least in the constricted section 43 and, in this embodiment, the first through hole 46 extends slightly beyond the constricted section 43 to the front and rear sections 41, 42. The first through hole 46 extends in the longitudinal direction Y so as to overlap the imaginary longitudinal center line P-P. In this embodiment, a dimension in the longitudinal direction Y of the first through hole 46 is about 180mm and a dimension in the transverse direction X is about 25mm. The first through hole 46 defines, on the upper surface 44, the upper opening including front and rear ends 46a, 46b and opposite side edges 46c and defines, in the thickness direction, a peripheral wall 46d.

The second layer element 50 has a substantially rectangular outer shape and has a dimension in the transverse direction X substantially equal to that of the constricted section 43 of the first layer element 40. The second layer element 50 has a lower surface 51 facing the backsheet 30 and an upper surface 52 facing the first layer element 40 and the second layer element 50 is formed with a slot-like second through hole 53 extending nearly vertically in the thickness direction from the upper surface 52 to the lower surface 51. The second through hole 53 extends in the longitudinal direction Y so as to overlap the imaginary longitudinal center line P-P and also to overlap the first through hole 46. The second through hole 53 defines, on the lower surface 51, the lower opening including front and rear ends 53a, 53b and opposite side edges 53c and defines, in the thickness direction, a peripheral wall 53d.

In this embodiment, a dimension in the longitudinal direction Y of the second through hole 53 is about 90mm and a dimension in the transverse direction X thereof is about 12mm. With such dimensioning, the first through hole 46 has an opening area larger than that of the second through hole 53 and, when the first layer element 40 is lapped over the second layer element 50, the opening of the second through hole 53 is exposed to the inside of the opening of the first through hole 46. In other words, the front and rear ends 53a, 53b and the opposite side edges 53c of the second through hole 53 extend inward from the front and rear ends 46a, 46b and the opposite side edges 46c of the first through hole 46 to form a stepped region 91 between the first through hole 46 and the second through hole 53.

The stepped region 91 is defined by the peripheral wall 46d of the first through hole 46, the upper surface of the second layer element 50 and the peripheral wall 53d of the second through hole 53. Specifically, the stepped region 91 includes a portion in which the first layer element 40 and the second layer element 50 overlap each other and a portion defined by the second layer element 50 only and a thickness difference resulting from such partial overlapping defines the stepped region 91. The stepped region 91 formed in this manner makes it possible to improve the absorption capacity for bodily fluids in comparison with the case in which the peripheral wall 46d and the peripheral wall 53d are aligned with each other in the thickness direction and extend almost vertically. More specifically, while the effect of bodily fluid-absorption obtained in the through holes is limited to the continuous peripheral wall of the first through hole 46 and the second through hole 53 if the opening area of the first through hole 46 is equal to the opening area of the second through hole 53, according to this embodiment, in contrast, bodily fluids may be absorbed not only along the respective peripheral walls 46d, 53d of the first through hole 46 and the second through hole 53 but also the upper surface 52 of the second layer element 50. In this way, it is possible to accelerate the absorption rate and thereby to improve the absorption capacity.

The above-mentioned first layer element 40 and second layer element 50 are respectively provided with compressed grooves 92 outboard of the first through hole 46 and the second through hole 53 as viewed in the transverse direction X. The compressed grooves 92 are formed by compressing from the upper surface 44 toward the lower surface 51 under heating the first layer element 40, the second layer element 50 and the liquid diffusion sheets 61, 62 stacked on each other. The compressed grooves 92 formed in this manner serve to tighten the stacking of the first layer element 40 and the second layer element 50 and, at the same time, increase the density of the first and second layer elements in the vicinity of the respective compressed grooves 92, so that bodily fluids such as urine may be smoothly guided from the upper surface 44 toward the lower surface 51. In addition, with the urine absorption pad 10 put on the wearer's body, the urine absorption pad 10 is easily folded along the compressed grooves 92. In this embodiment, a dimension in the longitudinal direction Y of the first through hole 46 is larger than a dimension in the longitudinal direction Y of the compressed grooves 92.

The second layer element 50 is bonded to the backsheet 30 by bonding means 31 such as hot melt adhesives by the intermediary of the second liquid diffusion sheet 62. Two or more these bonding means 31 extend in the longitudinal direction Y and are spaced apart from each other in the transverse direction X.

The urine absorption pad 10 as has been described above is preferably put on the wearer's body so that the first and second through holes 46, 53 may be aligned with the wearer's areas of excretion. The urine absorption pad 10 may be used in combination with a separately prepared diaper, for example, by attaching the urine absorption pad 10 to the inside of the diaper. It is also possible to use the urine absorption pad 10 by attaching it to the wearer's underwear or clothing. Urine discharged on the urine absorption pad 10 put on the wearer's body in such a manner permeates the topsheet 20, then is diffused by the first liquid diffusion sheet 61 in the longitudinal direction Y as well as in the transverse direction X and absorbed by the first layer element 40 and partially flows into the first through hole 46. Urine having flown into the first through hole 46 may be absorbed also through the stepped region 91 as has previously been described.

The second liquid diffusion sheet 62 is interposed between the first layer element 40 and the second layer element 50 and discharged urine is transferred from the first layer element 40 to the second layer element 50 along the second liquid diffusion sheet 62 and absorbed by the first layer element 40 or the second layer element 50. Urine having flown down along the first through hole 46 and the second through hole 53 is transferred to the boundary between the second layer element 50 and the backsheet 30 and may be absorbed by the second layer element 50 from the lower surface 51. The second liquid diffusion sheet 62 is interposed between the second layered region 50 and the backsheet 30 and therefore urine having attained this level is diffused along the second liquid diffusion sheet 62 in the longitudinal direction Y as well as in the transverse direction X and simultaneously absorbed by the second layer element 50.

As has been described above, interposition of the first and second liquid diffusion sheets 61, 62 makes it possible to utilize the liquid-absorbent core material over a wide range. In other words, it is possible to restrict the problem that the discharged urine might be locally absorbed by the absorbent member and, in consequence, the absorption rate might be lowered and the absorption region might be limited.

Fig. 5 is a diagram corresponding to Fig. 4 except that Fig. 5 illustrates the sectional diagram of Fig. 4 when the urine absorption pad 10 is put on the wearer ' s body. The urine absorption pad 10 is preferably put on the wearer's body so that the front section 41 of the first layer element 40 lies in a front waist side so as to cover the wearer's external genitalia and the rear section 42 lies in the wearer's rear waist side so as to cover the wearer's buttocks. With the urine absorption pad 10 put on the wearer's body in such a manner, the constricted section 43 is squeezed by the wearer's inguinal regions or thighs from outside toward inside in the transverse direction X. When the urine absorption pad 10 is squeezed in this manner, the cross-section thereof is folded in W-shape as illustrated in Fig. 5.

In the urine absorption pad 10, a stiffness difference between the compressed grooves 92 and the first and second through holes 46, 53 and the remaining region facilitates the formation of fold lines extending in parallel to the imaginary longitudinal center line P-P. In the midsection in the transverse direction X including the first and second through holes 46, 53, the pad is folded closer to the wearer's body and the compressed grooves 92 are folded so as to be spaced apart from the wearer's body. In this way, the cross-section of the pad 10 is folded in W-shape and further facilitates discharged urine to flow into the first and second through holes 46, 53.

The bonding means 31 are provided between the second layer element 50 and the backsheet 30 so as to be spaced apart from each other in the transverse direction X so that, between each pair of the bonding means 31, the second layer element 50 is spaced apart from the backsheet 30 to define a void space 93. These void spaces 93 may temporarily retain discharged urine. Even when superabsorbent polymer particles or the like having a relatively low initial absorption rate are used for the liquid-absorbent core material, such void spaces 93 ensure discharged urine is completely absorbed.

In the above-mentioned urine absorption pad 10, the stepped regions 91 define portions thinner than the remaining portion between the front and rear ends 46a, 46b of the first through hole 46 and the front and rear ends 53a, 53b of the second through hole 53. These thinner portions have a stiffness lower than that of the remaining portion and provide a relatively high flexibility facilitating folding of the pad along the aforementioned fold lines. Particularly in the state as illustrated in Fig. 5, such a high flexibility may prevent the opposite side edges 53c from coming in contact with each other and thereby closing this through hole 53. If the through hole 53 is closed, discharged urine may not flow downward to the lower surface 51 of the second layer element 50 and absorption of urine from the lower surface may not be expected. In contrast, the embodiment according to this invention may effectively overcome such a problem. In this regard, in order that the absorbent member may have an appropriate flexibility, the liquid-absorbent core material is preferably formed of pulp or the like having a relatively long fiber length.

In the constitution described above, the first and second layer elements 40, 50 may be formed by layering liquid-absorbent core material such as pulp fibers in the thickness direction. In this case, the first and second through holes 46, 53 may be formed by layering the core material so that none of the core material is stacked in regions corresponding to the first and second through holes 46, 53. Alternatively, it is also possible to layer the core material over respective whole areas corresponding to the first and second layer elements 40, 50 and thereafter to hollow out the first and second through holes 46, 53 from the respective layer elements 40, 50.

While the first and second liquid diffusion sheets 61, 62 are spaced apart from the respective peripheral walls 46d, 53d of the first and second through holes 46, 53 according to this embodiment, it is also possible to put these sheets 61, 62 in close contact with the respective peripheral walls 46d, 53d. By putting the first and second liquid diffusion sheets 61, 62 in close contact with the respective peripheral walls 46d, 53d, it is possible to improve urine absorption efficiency from the peripheral walls.

According to this embodiment, the second through hole 53 is positioned roughly in the center of the first through hole 46 so that the front and rear ends 53a, 53b and the opposite side edges 53c of the second through hole 53 extend inward from the front and rear ends 46a, 46b and the opposite side edges 46c of the first through hole 46 so that the stepped region 91 is formed fully around the peripheral walls 46d, 53d in the circumferential direction. In this regard, however, it is possible to form the stepped region 91 on any one of the front and rear ends 46a, 46b or to form along respective parts of the peripheral walls 46d and 53d in the circumferential direction. Specifically, an alternative arrangement is also possible such that the first and second through holes 46, 53 have the same dimension in the transverse direction X and the stepped region 91 is not formed in the transverse direction X. Furthermore, an alternative arrangement is also possible such that the second through hole 53 is eccentrically-located in the longitudinal direction Y of the first through hole 46 and the stepped region 91 is formed only one of the front and rear ends 46a, 46b. In addition, it is not essential that the second through hole 53 is positioned inside the first through hole 46 and one of the front and rear ends 53a, 53b may extend outward beyond the first through hole 46.

According to this embodiment, the first through hole 46 extends from the constricted section 43 extends to the front and rear sections 41, 42 and are dimensioned to be longer than the compressed grooves 92 in the longitudinal direction Y. The dimension in the longitudinal direction Y of the first and second through holes 46, 53 may be enlarged to enlarge the opening area on the upper surface 44 and thereby to absorb urine quickly, even if this amount is relatively large. Furthermore, the first and second layer elements 40, 50 are adequately long in the longitudinal direction Y and the first and second through holes 46, 53 also may be dimensioned adequately long in the longitudinal direction Y to utilize the liquid-absorbent core material of these first and second layer elements 40, 50 efficiently.

While the absorbent member has been described to include the first layer element 40 and the second layer element 50, this invention is not limited to such a constitution and it is possible for the absorbent member to include three or more layer elements. In such a case, opening areas of the respective through holes may be dimensioned to be stepwise or continuously reduced from the uppermost layer element to the lowermost layer element. While the first and second through holes 46, 53 are formed in front of the imaginary transverse center line Q-Q in this embodiment, this invention is not limited to such location of the through holes 46, 53 and the locations of these through holes 46, 53 may be appropriately selected. Particularly, both the dimensions and the locations of the first and second through holes may be selected depending on a whole length in the longitudinal direction Y of the urine absorption pad 10. The first and second through holes 46, 53 respectively have a dimension in the longitudinal direction Y larger than a dimension in the transverse direction X. Such dimensioning makes it possible to ensure an adequately large urine absorption region when the wearer is in a side lying posture. Specifically, urine flowing into the first and second through holes 46, 53 is diffused within these through holes and simultaneously permeates through a horizontal surface extending over a dimension corresponding to the dimension in the longitudinal direction Y. Therefore, the larger the dimension in the longitudinal direction, the larger the area of the permeation region.

### <Second Embodiment>

Fig. 6 is a plan view of the absorbent member according to a second embodiment. In this embodiment, a second through region 55 is characterized in that the second through hole 55 includes a trunk segment 56 extending on the imaginary longitudinal center line P-P and a pair of branch segments 57 extending forward in the longitudinal direction Y from the trunk segment 56 and diverging in the transverse direction X. The other constituents are similar to those in the first embodiment and will not be repetitively described hereunder.

The first layer element 40 and the first through hole 46 are similar to those in the first embodiment. The second through hole 55 formed in the second layer element 50 includes the trunk segment 56 extending in the longitudinal direction Y and a pair of branch segments 57 extending from the trunk segment 56 and is Y-shaped as a whole. The branch segments 57 diverge forward and are spaced apart from each other at an angle R of about 60°. Of such second through hole 55, the trunk segment 56 overlaps with the first through hole 46 and the branch segments 57 extend from the first through hole 46. In other words, the branch segments 57 lie outboard of the front end 46a of the first through hole 46 as viewed in the longitudinal direction Y.

In this embodiment, a stepped region 91 is formed between the rear end 46b of the first through hole 46 and the rear end 55b of the second through hole 55.

With a urine absorption pad 10 having the constitution as has been described above, the first and second layer elements 40, 50 are folded along the first and second through holes 46, 55 in the same manner as in the first embodiment. In the second through hole 55, the urine absorption pad 10 is folded along the trunk segment 56 and the branch segments 57. The branch segments 57 are located in the front waist side and therefore the urine absorption pad 10 can be put in surface-contact with the wearer's body particularly in the vicinity of excretion. In this way, the urine absorption pad 10 may be put in close contact with the wearer's body over a sufficiently large area for urine absorption.

The second through hole 55 lying outboard of the front end 46a of the first through hole 46 in the longitudinal direction Y makes it possible to diffuse discharged urine over a correspondingly enlarged area before absorption, without reducing the absorption capacity of the first through hole 46. This is for the reason that, while the opening area of the first through hole 46 is not changed, urine flowing into the first through hole 46 may be diffused farther via the trunk segment 56 and the branch segments 57 of the second through hole 55.

While the branch segments 57 extend forward from the trunk segment 56 in this embodiment, it is possible to form the branch segments so as to extend rearward. Dimensions of the trunk segment 56 and the branch segments 57 as well as the relative positions thereof to the first through hole 46 are not limited to this embodiment and may be appropriately varied.

### {Reference Signs List}

- 10: urine absorption pad (absorbent article)
- 20: topsheet
- 30: backsheet
- 31: bonding means
- 40: first layer element
- 44: upper surface
- 46: first through hole
- 46a: front end
- 46b: rear end
- 46d: peripheral wall
- 50: second layer element
- 51: lower surface
- 53: second through hole
- 53a: front end
- 53b: rear end
- 53d: peripheral wall
- 55: second through hole
- 61: first liquid diffusion sheet
- 62: second liquid diffusion sheet
- 91: stepped region
- 92: compressed groove
- X: transverse direction
- Y: longitudinal direction

## Claims

1. An absorbent article (10) having a longitudinal direction (Y) and a transverse direction (X), the article (10) including:
a topsheet (20) lying on a side facing the wearer's body;
a backsheet (30) lying on a side opposite to the side facing the wearer's body; and
an absorbent member (40, 50) interposedbetweenthetopsheet (20) and the backsheet (30); wherein
the absorbent member (40, 50) is formed with a through hole extending through the absorbent member (40, 50) from an upper surface (44) thereof lying on the side of the topsheet (20) to a lower surface (51) thereof lying on the side of the backsheet (30), wherein:
the through hole has an upper opening (46) on the upper surface (44), a lower opening (53) on the lower surface (51) and a peripheral wall (46d, 53d) connecting the upper opening to the lower opening; and
at least one of front (46a) and rear (46b) ends in the longitudinal direction (Y) of the upper opening is not aligned in a thickness direction (X) with the front (53a) and rear (53b) ends in the longitudinal direction (Y) of the lower opening and the peripheral wall (46d, 53d) extends down- and inward from the front (46a) and/or rear (46b) end in the longitudinal direction (Y) of the upper opening to the front (53a) and/or rear (53b) end of the lower opening; wherein
upper (44) and lower (51) surfaces of the absorbent member (40, 50) are covered with liquid diffusion sheets (61), respectively; wherein
the absorbent member includes a plurality of layer elements (40, 50) stacked in the thickness direction and a liquid diffusion sheet (62) is interposed between each pair of the adjacent layer elements (40, 50); wherein
the absorbent member (40, 50) is formed with compressed grooves (92) extending in the longitudinal direction (Y) outboard of the through hole as viewed in the transverse direction (X).
**characterized in that**:
between the lower surface of the absorbent member (51) and the backsheet (30), there are provided two or more bonding means (31) spaced apart from each other in the transverse direction (X) and extending in a longitudinal direction (Y).

2. The absorbent article (10) defined by Claim 1, wherein an opening area of the upper opening is larger than that of the lower opening and a dimension in the longitudinal direction (Y) of the upper opening is larger than that of the lower opening.

3. The absorbent article (10) defined by Claim 1 or 2, wherein one of the front (46a,53a) and rear (46b, 53b) ends of the lower opening extends outward in the longitudinal direction (Y) beyond the corresponding end of the upper opening.

4. The absorbent article (10) defined by any one of Claims 1 through 3, wherein the through hole has a dimension in the longitudinal direction (Y) larger than the dimension thereof in the transverse direction (X).

5. The absorbent article (10) defined by any one of Claims 1 through 4, wherein the peripheral wall (46d,53d) is formed with a stepped region (91) created by a thickness dimension difference of the peripheral wall (46d,53d) and extending in a circumferential direction.

## Patentansprüche

1. Absorbierender Artikel (10), der eine Längsrichtung (Y) und eine Querrichtung (X) aufweist, wobei der Artikel (10) Folgendes einschließt:
eine obere Lage (20), die auf einer Seite liegt, die dem Körper des Trägers zugewandt ist;
eine rückseitige Lage (30), die auf einer Seite liegt, die der Seite gegenüberliegt, die dem Körper des Trägers zugewandt ist; und
ein absorbierendes Element (40, 50), das zwischen die obere Lage (20) und die rückseitige Lage (30) eingefügt ist; wobei
das absorbierende Element (40, 50) mit einem Durchgangsloch ausgebildet ist, das sich durch das absorbierende Element (40, 50) von einer oberen Oberfläche (44) davon, die auf der Seite der oberen Lage (20) liegt, zu einer unteren Oberfläche (51) davon, die auf der Seite der rückseitigen Lage (30) liegt, erstreckt, wobei:
des Durchgangsloch Folgendes aufweist: eine obere Öffnung (46) auf der oberen Oberfläche (44), eine untere Öffnung (53) auf der unteren Oberfläche (51) und eine umlaufende Wand (46d, 53d), die die obere Öffnung mit der unteren Öffnung verbindet; und
mindestens eines von vorderen (46a) und hinteren (46b) Enden in der Längsrichtung (Y) der oberen Öffnung in der Dickenrichtung (X) nicht mit den vorderen (53a) und hinteren (53b) Enden in der Längsrichtung (Y) der unteren Öffnung ausgerichtet ist und sich die umlaufende Wand (46d, 53d) nach unten und nach innen von dem vorderen (46a) und/oder hinteren (46b) Ende in der Längsrichtung (Y) der oberen Öffnung zu dem vorderen (53a) und/oder hinteren (53b) Ende der unteren Öffnung erstreckt; wobei
obere (44) und untere (51) Oberflächen des absorbierenden Elements (40, 50) jeweils mit Flüssigkeitsdiffusionslagen (61) bedeckt sind; wobei
das absorbierende Element eine Vielzahl von Schichtelementen (40, 50) einschließt, die in der Dickenrichtung geschichtet sind, und eine Flüssigkeitsdiffusionslage (62) zwischen jedes Paar der benachbarten Schichtelemente (40, 50) eingefügt ist; wobei
das absorbierende Element (40, 50) mit komprimierten Rillen (92) ausgebildet ist, die sich in der Längsrichtung (Y) außenliegend von dem Durchgangsloch, bei Betrachtung in der Querrichtung (X), erstrecken;
**dadurch gekennzeichnet, dass**:
zwischen der unteren Oberfläche des absorbierenden Elements (51) und der rückseitigen Lage (30) zwei oder mehr Bindungsmittel (31) bereitgestellt sind, die in der Querrichtung (X) voneinander beabstandet sind und sich in einer Längsrichtung (Y) erstrecken.

2. Absorbierender Artikel (10), wie durch Anspruch 1 definiert, wobei eine Öffnungsfläche der oberen Öffnung größer ist als die der unteren Öffnung und eine Abmessung in der Längsrichtung (Y) der oberen Öffnung größer ist als die der unteren Öffnung.

3. Absorbierender Artikel (10), wie durch Anspruch 1 oder 2 definiert, wobei sich eines der vorderen (46a, 53a) und hinteren (46b, 53b) Enden der unteren Öffnung nach außen in der Längsrichtung (Y) über das entsprechende Ende der oberen Öffnung hinaus erstreckt.

4. Absorbierender Artikel (10), wie durch einen der Ansprüche 1 bis 3 definiert, wobei das Durchgangsloch eine Abmessung in der Längsrichtung (Y) aufweist, die größer ist als die Abmessung davon in der Querrichtung (X).

5. Absorbierender Artikel (10), wie durch einen der Ansprüche 1 bis 4 definiert, wobei die umlaufende Wand (46d, 53d) mit einem stufenförmigen Bereich (91) ausgebildet ist, der durch einen Abmessungsunterschied der Dicke der umlaufenden Wand (46d, 53d) erzeugt wird und sich in einer Umfangsrichtung erstreckt.

## Revendications

1. Article absorbant (10) ayant une direction allant dans le sens longitudinal (Y) et une direction allant dans le sens transversal (X), l'article (10) comprenant :
une feuille de dessus (20) reposant sur un côté orienté vers le corps de l'utilisateur ;
une feuille de support (30) reposant sur un côté opposé au côté orienté vers le corps de l'utilisateur ; et
un élément absorbant (40, 50) intercalé entre la feuille de dessus (20) et la feuille de support (30) ; dans lequel
l'élément absorbant (40, 50) est formé avec un trou traversant s'étendant au travers de l'élément absorbant (40, 50) depuis une surface supérieure (44) de celui-ci reposant sur le côté de la feuille de dessus (20) jusqu'à une surface inférieure (51) de celui-ci reposant sur le côté de la feuille de support (30), dans lequel :
le trou traversant a une ouverture supérieure (46) sur la surface supérieure (44), une ouverture inférieure (53) sur la surface inférieure (51) et une paroi périphérique (46d, 53d) reliant l'ouverture supérieure à l'ouverture inférieure ; et
au moins l'une parmi les extrémité avant (46a) et arrière (46b) dans la direction allant dans le sens longitudinal (Y) de l'ouverture supérieure n'est pas alignée dans une direction allant dans le sens de l'épaisseur (X) sur les extrémités avant (53a) et arrière (53b) dans la direction allant dans le sens longitudinal (Y) de l'ouverture inférieure et la paroi périphérique (46d, 53d) s'étend vers le bas et vers l'intérieur depuis l'extrémité avant (46a) et/ou arrière (46b) dans la direction allant dans le sens longitudinal (Y) de l'ouverture supérieure jusqu'à l'extrémité avant (53a) et/ou arrière (53b) de l'ouverture inférieure ; dans lequel
les surfaces supérieure (44) et inférieure (51) de l'élément absorbant (40, 50) sont recouvertes de feuilles de diffusion des liquides (61), respectivement ; dans lequel
l'élément absorbant comprend une pluralité d'éléments de couche (40, 50) empilés dans la direction allant dans le sens de l'épaisseur et une feuille de diffusion des liquides (62) est intercalée entre chaque paire d'éléments de couche adjacents (40, 50) ; dans lequel
l'élément absorbant (40, 50) est formé avec des rainures comprimées (92) s'étendant dans la direction allant dans le sens longitudinal (Y) du côté extérieur du trou traversant quand vu dans la direction allant dans le sens transversal (X),
**caractérisé en ce que** :
entre la surface inférieure (51) de l'élément absorbant et la feuille de support (30), sont mis en oeuvre deux ou plusieurs moyens de liaison (31) espacés les uns des autres dans la direction allant dans le sens transversal (X) et s'étendant dans une direction allant dans le sens longitudinal (Y).

2. Article absorbant (10) selon la revendication 1, dans lequel une zone d'ouverture de l'ouverture supérieure est plus grande que celle de l'ouverture inférieure et une dimension dans la direction allant dans le sens longitudinal (Y) de l'ouverture supérieure est plus grande que celle de l'ouverture inférieure.

3. Article absorbant (10) selon la revendication 1 ou la revendication 2, dans lequel l'une des extrémités avant (46a, 53a) et arrière (46b, 53b) de l'ouverture inférieure s'étend vers l'extérieur dans la direction allant dans le sens longitudinal (Y) au-delà de l'extrémité correspondante de l'ouverture supérieure.

4. Article absorbant (10) selon l'une quelconque des revendications 1 à 3, dans lequel le trou traversant a une dimension dans la direction allant dans le sens longitudinal (Y) plus grande que la dimension de celui-ci dans la direction allant dans le sens transversal (X).

5. Article absorbant (10) selon l'une quelconque des revendications 1 à 4, dans lequel la paroi périphérique (46d, 53d) est formée avec une région étagée (91) créée par une différence de dimension d'épaisseur de la paroi périphérique (46d, 53d) et s'étendant dans une direction allant dans le sens de la circonférence.
